# EUROPEAN PATENT SPECIFICATION

(11) **EP 1 739 086 B1**
(45) Date of publication and mention of the grant of the patent: **04.03.2009**
(21) Application number: 06386012.6
(22) Date of filing: 24.05.2006
(51) Int. Cl.: C07D 413/12, A61K 9/08, A61K 31/519, C07D 413/14

(54) **Pharmaceutical compositions of risperidone in aqueous solution**
Pharmazeutische Zusammensetzungen von Risperidon in wässriger Lösung
Compositions pharmaceutiques de risperidone en solution aqueuse

(30) Priority: 29.06.2005 GR 2005100326
(43) Date of publication of application: 03.01.2007
(73) Proprietor: Verisfield (UK) Ltd., 115 28 Athens (GR)
(72) Inventor: Avgoustakis, Konstantinos, 26 442 Patras (GR)

(56) References cited:
- EP-A1- 0 005 768
- EP-B1- 0 769 965
- GB-A- 1 525 765

## Description

### Introduction

The present invention is concerned with a new composition for the production of stable and high viscosity aqueous solutions of Risperidone, for oral administration.

### Background of the invention

Risperidone is a strong antipsychotic agent, which is widely used in the treatment of psychotic disorders.

EP 0, 196, 132 (1984) discloses the production of unbuffered aqueous solutions of Risperidone, for oral administration, which contain polyhydric alcohols, glycerol and sorbitol, preservatives such as methyl paraben and propyl paraben, sweetening agents such as saccharin sodium and flavoring agents (cherry flavor, raspberry flavor) and a small amount of water. Moreover, it discloses the production of oral drops containing Risperidone as the active ingredient and the use of lactic acid, sweetening and flavoring agents and a small quantity of water in propylene glycol. Finally it discloses the production of an unbuffered solution for injection of Risperidone, which makes use of preservatives like methyl paraben and propyl paraben, lactic acid and propylene glycol.

Sorbitol and the other polyhydric alcohols have been found to reduce the stability of the aqueous solutions of Risperidone, by inducing its decomposition.

The patents EP 0,769, 965 B1 (1997) and USP 5, 616 587 (1997) of Janssen disclose the production of aqueous solutions of Risperidone for oral and parenteral administration, using a buffering agent (more preferred the use of tartaric acid /sodium hydroxide) in order to maintain pH between 2 and 6. The solution is free of sorbitol or other polyhydric alcohols. The improved physicochemical stability of the product is revealed from the experimental data given in various storage temperatures.

The patent WO 2004017975 of Ranbaxy, discloses the production of stable aqueous solutions of Risperidone, using polyhydric alcohols, by reducing the solid content and increasing the water concentration of the aqueous solution. The stability may further be improved by incorporating small amounts of an antioxidant.

The stability of the aqueous solution of Risperidone was confirmed by the accelerated stability data generated at 80°C over a period of 4 weeks.

The use of polyhydric alcohols in the composition of the aqueous solution of the above mentioned patent arises the issue concerning the stability of the aqueous solutions of Risperidone in the presence of polyhydric alcohols (sorbitol), as the stability of the products has not been confirmed / tested for a long period of time.

### Description of the invention

The present invention concerns the composition of a stable aqueous oral solution of the antipsychotic agent, Risperidone, using amino acids such as glutamic acid or glycine and polymers such as hydroxypropyl-methylcellulose (HPMC), for the production of stable, unbuffered, aqueous and high viscosity solutions of Risperidone.

Risperidone is slightly soluble in water and the production of aqueous solutions having Risperidone as the active ingredient, is very difficult. In the present invention Risperidone is used in concentration (0,1-2) mg/ml, along with amino acids like glutamic acid and glycine in content between 0,5 and 2,0 w/v, for the production of aqueous solutions of Risperidone, where no buffering agent is needed in order to maintain pH within limits.

The amino acids, which are used in the present invention, are components of everyday's alimentation and natural components of the human body. Their use in the composition of the aqueous solution of Risperidone, eliminates / substitutes the need for buffering agents in order to maintain pH within desirable limits.

Moreover, the use of polyhydric alcohols (sorbitol, mannitol, propylene glycol e.t.c.) is excluded, as these components have been found to decompose Risperidone in solution.

According to the present invention, the composition also contains common excipients for the preparation of oral solutions such as hydroxypropyl-methylcellulose (HPMC) or hydroxypropyl-cellulose (HPC), in rate between 0,01% and 1,0% w/v, in order to increase the viscosity of the solution and to make oral administration easier.

The final composition of the oral solution also contains other classic excipients like the use of an antimicrobial agent (preferably benzoic acid) and the use of sweetening and flavoring agents.

The optimum antimicrobial action of benzoic acid in aqueous solutions depends on the pH range of the preparations of the present invention. The optimum antimicrobial action of benzoic acid, according to the international bibliography, appears in solutions having pH ranging between 2,5 and 4,5.

The pH of the aqueous oral solutions of the present invention range between 3.3 and 3.8 for the preparations that contain glutamic acid and between 3.9 and 4.4 for the preparations that contain glycine.

The present invention uses the following ingredients:

### 1) Oral solution

a. Risperidone, as the active ingredient.
b. Glutamic acid or Glycine, as regulators / stabilizing agents of the solution.
c. Hydroxypropyl-methylcellulose (HPMC) or Hydroxypropyl-cellulose (HPC), as agents which increase the viscosity of the aqueous solution.
d. An antimicrobial agent, preferably benzoic acid, due to the pH range of the described preparations.
e. Saccharin sodium, as a sweetening agent.
f. Cherry flavor or Caramel flavor, as flavoring agents

### Preparation of the oral solution

a) All the required quantities of hydroxypropyl-methylcellulose (HPMC) or hydroxypropyl-cellulose (HPC) are weighed and transferred in the preparation tank, which is equipped with a stirrer. Half of the total required quantity of the water is added and the mixture is left under stirring, in a covered container, for approximately 20 hours, until hydroxypropyl-methylcellulose (HPMC) or hydroxypropyl-cellulose (HPC) is completely dissolved.
b) To the above solution, the required quantities of benzoic acid and glutamic acid or glycine are added. Then, the mixture is heated at 70-80 °C, under stirring, until all the ingredients are completely dissolved.
c) To the above solution, the entire quantity of Risperidone is added under stirring. Stirring should be continued until Risperidone is totally dissolved (approximately 2 hours).
d) When Risperidone is completely dissolved, the solution is left to cool at room temperature and then saccharin sodium is added under stirring.
e) The solution is left under stirring for 5-10 minutes.
f) Then, the flavoring agents are added (cherry flavor or caramel flavor), under stirring.
g) Finally, the remaining quantity of the water is added, under stirring.
Filtration through filter-press follows in final step.

### Example 1

Required quantities per 1 ml of oral solution

| Component | Quantity | Quantity | Quantity |
|---|---|---|---|
| Risperidone | 2 mg | 2 mg | 2 mg |
| Glutamic acid | 7,5 mg | 7,5 mg | 7,5 mg |
| Hydroxypropyl-methylcellulose | 5 mg | 5 mg | 5 mg |
| Benzoic acid | 2 mg | 2 mg | 2 mg |
| Saccharin sodium | 1 mg | 1 mg | - |
| Water | qs 1 ml | qs 1 ml | qs 1 ml |
| Cherry flavor | 0,75 mg | - | - |
| Caramel flavor | 0,30 mg | - | - |

### Example 2

Required quantities per 1 ml of oral solution

| Component | Quantity | Quantity | Quantity |
|---|---|---|---|
| Risperidone | 2 mg | 2 mg | 2 mg |
| Glutamic acid | 7,5 mg | 7,5 mg | 7,5 mg |
| Hydroxypropyl-cellulose | 5 mg | 5 mg | 5 mg |
| Benzoic acid | 2 mg | 2 mg | 2 mg |
| Saccharin sodium | 1 mg | 1 mg | - |
| Water | qs 1 ml | qs 1 ml | qs 1 ml |
| Cherry flavor | 0,75 mg | - | - |
| Caramel flavor | 0,30 mg | - | - |

### Example 3

Required quantities per 1 ml of oral solution

| Component | Quantity | Quantity | Quantity |
|---|---|---|---|
| Risperidone | 2 mg | 2 mg | 2 mg |
| Glycine | 7,5 mg | 7,5 mg | 7,5 mg |
| Hydroxypropyl-methylcellulose | 5 mg | 5 mg | 5 mg |
| Benzoic acid | 2 mg | 2 mg | 2 mg |
| Saccharin sodium | 1 mg | 1 mg | - |
| Water | qs 1 ml | qs 1 ml | qs 1 ml |
| Cherry flavor | 0,75 mg | - | - |
| Caramel flavor | 0,30 mg | - | - |

### Example 4

Required quantities per 1 ml of oral solution

| Component | Quantity | Quantity | Quantity |
|---|---|---|---|
| Risperidone | 2 mg | 2 mg | 2 mg |
| Glycine | 7,5 mg | 7,5 mg | 7,5 mg |
| Hydroxypropyl-cellulose | 5 mg | 5 mg | 5 mg |
| Benzoic acid | 2 mg | 2 mg | 2 mg |
| Saccharin sodium | 1 mg | 1 mg | - |
| Water | qs 1 ml | qs 1 ml | qs 1 ml |
| Cherry flavor | 0,75 mg | - | - |
| Caramel flavor | 0,30 mg | - | - |

## Claims

1. Composition of a stable aqueous solution of Risperidone, for oral administration, with the characteristic that it contains amino acids as regulators and stabilizing agents and celluloses as agents that increase the viscosity of the solution, which also excludes the use of polyhydric alcohols.

2. Composition of a stable aqueous solution of Risperidone according to claim 1, which contains Risperidone ranging between 0,1 mg/ml and 2 mg/ml.

3. Composition of a stable aqueous solution of Risperidone according to claim 1, where the concerned amino acids are glutamic acid or glycine.

4. Composition of a stable aqueous solution of Risperidone according to claim 3, which contains glutamic acid ranging between 0,5% and 2,0% (w/v).

5. Composition of a stable aqueous solution of Risperidone according to claim 4 having pH ranging between 3.3 and 3.8 which is suitable for oral administration.

6. Composition of a stable aqueous solution of Risperidone according to claim 3 which contains glycine ranging between 0,5% and 2,0% (w/v).

7. Composition of a stable aqueous solution of Risperidone according to claim 6 having pH ranging between 3.9 and 4.4 which is suitable for oral administration.

8. Composition of a stable aqueous solution of Risperidone according to claim 1, which contains hydroxypropyl-methylcellulose or hydroxypropyl-cellulose as agents that increase the viscosity of the aqueous solution.

9. Composition of a stable aqueous solution of Risperidone according to claim 8, which contains Risperidone ranging between 0,1 mg/ml and 2 mg/ml, glutamic acid or glycine ranging between (0,5 - 1,0)% w/v, hydroxypropyl-methylcellulose or hydroxypropyl-cellulose ranging between (0,01 - 1,0)% w/v, an antimicrobial agent and water.

10. Composition of a stable aqueous solution of Risperidone according to claim 9, which contains one or more sweetening agents and/or one ore more, flavoring agents.

## Patentansprüche

1. Zusammenstellung eines stabilen wässrige lösung von risperidone zur darreichung per os, zeichnet sich durch die tatsache, dass enthält aminosäuren als faktoren zur regelung und stabilisierung, plus verwendung cellulose als faktoren erhöhen die viskosität der lösung und ist frei von mehrwertige alkohole.

2. Zusammenstellung eines stabilen wässrige lösung von risperidone gemäß anspruch 1, die enhält risperidone zwischen 0,1 mg/ml und 2 mg/ml.

3. Zusammenstellung eines stabilen wässrige lösung von risperidone gemäß anspruch 1, in dem die gemeldete aminosäuren sind glutaminsäure oder glycin.

4. Zusammenstellung eines stabilen wässrige lösung von risperidone gemäß anspruch 3, die enthält glutaminsäure zwischen 0,5% und 2,0% (w / v).

5. Zusammenstellung eines stabilen wässrige lösung von risperidone gemäß anspruch 4, der hat einen pH-Wert zwischen 3,3 und 3,8 und eignet sich zur darreichung per os.

6. Zusammenstellung eines stabilen wässrige lösung von risperidone gemäß anspruch 3, die enthält glycin zwischen 0,5% und 2,0% (w / v).

7. Zusammenstellung eines stabilen wässrige lösung von risperidone gemäß anspruch 6, der hat einen pH-Wert zwischen 3,9 und 4,4 und eignet sich zur darreichung per os.

8. Zusammenstellung eines stabilen wässrige lösung von risperidone gemäß anspruch 1, die enhält hydroxypropyl methylcellulose oder hydroxypropyl cellulose als Faktoren, die erhöhen die viskosität der wässrigen lösung.

9. Zusammenstellung eines stabilen wässrige lösung von risperidone gemäß anspruch 8, die enhält risperidone zwischen 0,1 mg/ml und 2 mg/ml, glutaminsäure oder glycin zwischen (0,5 - 1,0)% w/v, hydroxypropyl methylcellulose oder hydroxypropyl cellulose zwischen (0,01 - 1,0)% w/v, ein antimikrobenmittel und wasser.

10. Zusammenstellung eines stabilen wässrige lösung von risperidone gemäß anspruch 9, die enhält ein oder mehrere Süßungsmittel und / oder ein oder mehrere geschmacksrichtungen.

## Revendications

1. Composition d' une solution aqueuse stable de rispéridone, à administrer per os, **caractérisée par** sa teneur en acides aminés comme facteurs de régulation et de stabilisation, qui de plus utilise des celluloses comme facteurs d' augmentation de la viscosité de la solution et est dépourvue d' alcools polyhydroxyliques.

2. Composition d' une solution aqueuse stable de rispéridone, conformément à la revendication 1, qui contient une quantité de rispéridone, entre 0,1 mg/ml et 2 mg/ml.

3. Composition d' une solution aqueuse stable de rispéridone, conformément à la revendication 1, dans laquelle les acides aminés concernés sont l'acide glutamique ou la glycine.

4. Composition d' une solution aqueuse stable de rispéridone, conformément à la revendication 3, qui contient de l' acide glutamique, à un pourcentage entre 0,5% et 2,0% (poids / volume).

5. Composition d' une solution aqueuse stable de rispéridone, conformément à la revendication 4, ayant un pH entre 3,3 et 3,8 qui est appropriée pour l' administration per os.

6. Composition d' une solution aqueuse stable de rispéridone, conformément a la revendication 3, qui contient de la glycine à un pourcentage entre 0,5% et 2,0% (poids / volume).

7. Composition d' une solution aqueuse stable de rispéridone, conformément à la revendication 6, ayant un pH entre 3,9 et 4,4 qui est appropriée pour l' administration per os.

8. Composition d' une solution aqueuse stable de rispéridone, conformément à la revendication 1, qui contient de l' hydroxypropyl-méthyl-cellulose ou de l' hydroxypropyl-cellulose comme facteurs d' augmentation de la viscosité de la solution aqueuse.

9. Composition d' une solution aqueuse stable de rispéridone, conformément à la revendication 8, qui contient de la rispéridone entre 0,1 mg/ml et 2 mg/ml, de l' acide glutamique ou de la glycine entre (0,5 et 1,0)% poids / volume, de l' hydroxypropyl-méthyl-cellulose ou de l' hydroxypropyl-cellulose HPC entre (0,01 et 1,0) % poids / volume, d' un conservateur et de l'eau.

10. Composition d' une solution aqueuse de rispéridone, conformément à la revendication 9, qui contient une ou plusieurs facteurs édulcorant et/ou une ou plusieurs substances aromatiques.
